(19) Europäisches Patentamt
European Patent Office
Office européen des brevets

(11) Numéro de publication : **0 076 220**
**B1**

(12) **FASCICULE DE BREVET EUROPÉEN**

(45) Date de publication du fascicule du brevet : **07.08.85**

(21) Numéro de dépôt : **82420130.5**

(22) Date de dépôt : **23.09.82**

(51) Int. Cl.⁴ : **C 07 C 39/04**, C 07 C 39/08, C 07 C 39/28, C 07 C 43/23, C 07 C 37/055, C 07 C 41/26

(54) **Procédé d'hydrolyse d'éthers aryl-aliphatiques.**

(30) Priorité : **29.09.81 FR 8118572**

(43) Date de publication de la demande : **06.04.83 Bulletin 83/14**

(45) Mention de la délivrance du brevet : **07.08.85 Bulletin 85/32**

(84) Etats contractants désignés : **AT BE CH DE GB IT LI LU NL SE**

(56) Documents cités :
**DE-C- 730 236**
**US-A- 2 697 732**
**US-A- 2 878 292**
**US-A- 3 413 341**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Ratton, Serge**
**Vaulx Milieu**
**F-38290 La Verpilliere (FR)**

(74) Mandataire : **Vignally, Noel et al**
**RHONE-POULENC RECHERCHES Service Brevets Chimie et Polymères Centre de recherches de Saint-Fons B.P. 62**
**F-69192 St-Fons Cedex (FR)**

**EP 0 076 220 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un nouveau procédé d'hydrolyse d'au moins une fonction éther d'éthers aryl-aliphatiques.

On a décrit dans le brevet américain n° 2 878 292 la déméthylation de la vanilline par un agent tel que les acides chlorhydrique, bromhydrique, iodhydrique, le chlorhydrate d'aniline, ou d'éthanolamine, les halogénures d'aluminium, de zinc, de fer, d'étain, d'antimoine et de bore.

Mais ces agents de déméthylation sont en général très corrosifs pour l'appareillage, sont parfois très coûteux et posent souvent de délicats problèmes de traitement des masses réactionnelles finales.

Il a maintenant été trouvé de manière inattendue que l'on pouvait réaliser l'hydrolyse par l'eau d'au moins une fonction éther d'un éther aryl-aliphatique en opérant en présence d'un sel acide carboxylique.

Plus précisément le procédé selon l'invention consiste à hydrolyser au moins une fonction éther d'un composé de formule générale (I) :

$$R_1O—Ar—(R_2)_n \qquad (I)$$

dans laquelle

— Ar représente un cycle benzénique ou un cycle naphtalénique,

— $R_1$ représente :

un radical alkyle linéaire ou ramifié ayant de 1 à 4 atomes de carbone,

un radical alkényle linéaire ou ramifié ayant 3 ou 4 atomes de carbone,

— Les substituants $R_2$ identiques ou différents, représentent :

un groupement hydroxyle ;

un radical $OR_1$, $R_1$ ayant les significations indiquées précédemment ;

un radical alkyle linéaire ou ramifié ayant de 1 à 6 atomes de carbone ;

un radical alkényle linéaire ou ramifié ayant de 2 à 6 atomes de carbone ;

un radical phényle ;

un radical cycloalkyle ;

un radical phénylalkyle dans lequel la chaîne aliphatique comporte de 1 à 4 atomes de carbone ;

un radical cycloalkylalkyle dans lequel la chaîne aliphatique comporte de 1 à 4 atomes de carbone ;

un atome d'halogène ;

un groupement nitro ;

un groupement amine ;

un groupement aldéhyde —CHO ;

un groupement nitrile ;

un groupement hydroxycarbonyle ;

un groupement acyle de formule $—CO—R_3$ dans laquelle $R_3$ est un radical alkyle ayant de 1 à 6 atomes de carbone, cycloalkyle ayant 5 ou 6 atomes de carbone, phényle ou un enchaînement de plusieurs de ces radicaux ;

un groupement alkoxycarbonyle de formule $—COOR_4$ dans laquelle $R_4$ est un radical alkyle ayant de 1 à 6 atomes de carbone, alkényle ayant de 2 à 6 atomes de carbone, cycloalkyle ayant 5 ou 6 atomes de carbone, phényle ou un enchaînement de plusieurs de ces radicaux ;

— n est un nombre entier de 0 à 5,

ledit procédé étant caractérisé en ce que l'on opère en présence d'eau et en présence d'une quantité efficace d'un sel d'acide carboxylique choisi parmi les carboxylates d'un métal alcalin, d'ammonium ou d'un métal alcalino-terreux.

A titre d'exemples, on peut citer notamment les carboxylates de sodium, de potassium, de lithium et d'ammonium ; on peut également citer les carboxylates de calcium, de magnésium et de baryum. Ce sont cependant de préférence les carboxylates de métaux alcalins.

Les acides carboxyliques servant à l'obtention de tels carboxylates peuvent être les acides mono- ou polyfonctionnels, aliphatiques saturés ou insaturés, aromatiques, arylaliphatiques, cycloaliphatiques dont les cycles peuvent être substitués par un ou plusieurs radicaux.

On peut citer à titre d'illustration des acides carboxyliques précédents des monoacides aliphatiques saturés comme les acides acétique, propanoïque, n-butanoïque, méthyl-2 propanoïque, n-pentanoïque, méthyl-2 butanoïque, méthyl-3 butanoïque, diméthyl-3,3 butanoïque, n-hexanoïque, méthyl-2 pentanoïque, méthyl-3 pentanoïque, méthyl-4 pentanoïque ; des monoacides aliphatiques insaturés comme les acides propénoïque, butène-2 oïque, méthyl-2 propénoïque, butène-3 oïque, méthyl-2 butène-2 oïque cis (acide angélique), méthyl-2 butène-2 oïque trans (acide tiglique), pentène-4 oïque. hexène-3 oïque, hexène-4 oïque ; des diacides aliphatiques saturés comme l'acide malonique, l'acide succinique, l'acide glutarique, l'acide adipique ; des diacides éthyléniques comme l'acide maléique et l'acide fumarique ; des mono- ou des diacides aromatiques comme l'acide benzoïque, l'acide orthophtalique, l'acide isophtalique, l'acide téraphtalique, les acides mononitrobenzoïques, les acides monochlorobenzoïques ; des acides arylaliphatiques comme l'acide phénylacétique, les acides phényl-2 propanoïque, phényl-4 propanoïque ; des acides cycloaliphatiques comme l'acide cyclohexanedicarboxylique-1,3, l'acide cyclohexanedicarboxylique-1,4.

Parmi tous les carboxylates que l'on peut utiliser on préfère généralement les sels des acides carboxyliques aliphatiques saturés, monofonctionnels ayant de 2 à 6 atomes de carbone, tels que notamment l'acide acétique, les acides propanoïque, n-butanoïque, n-pentanoïque, n-hexanoïque, méthyl-2 propanoïque, méthyl-2 butanoïque, méthyl-3 butanoïque, diméthyl-3,3 butanoïque, méthyl-2 pentanoïque, méthyl-3 pentanoïque et méthyl-4 pentanoïque, ou difonctionnels ayant 3 à 6 atomes de carbone, tels que notamment l'acide malonique, l'acide succinique, l'acide glutarique et l'acide adipique, les sels de l'acide benzoïque et ceux des acides orthophtalique, isophtalique et téréphtalique. Parmi les sels de ces acides on préfère les sels de métaux alcalins.

De manière encore préférée, on emploie les sels de sodium ou de potassium de ces acides. Parmi ces derniers sels, on utilise préférentiellement l'acétate de sodium, le propionate de sodium et le succinate de sodium.

La quantité de sel d'acide carboxylique présent dans le milieu peut varier dans de larges limites. Si on exprime cette quantité par rapport à l'éther aryl-aliphatique, elle n'est généralement pas inférieure à 0,01 fois le poids dudit éther. La quantité maximale n'est pas critique. Habituellement elle ne dépasse pas 50 fois le poids d'éther. On préfère le plus souvent utiliser des rapports pondéraux sel d'acide carboxylique/éther aryl-aliphatique variant de 0,05 à 20.

Les éthers aryl-aliphatiques auxquels s'applique plus particulièrement le procédé selon l'invention sont des composés de formule générale (I) dans laquelle :

— Ar est un cycle benzénique ou naphtalénique,

— $R_1$ représente :

un radical alkyle linéaire ou ramifié ayant de 1 à 4 atomes de carbone tel que méthyle, éthyle, isopropyle, n-propyle, n-butyle, isobutyle ou tertiobutyle ;

un radical alkényle linéaire ou ramifié ayant 3 ou 4 atomes de carbone tel que allyle, propène-1-yle, isopropényle, butène-1-yle, butène-2-yle, butène-3-yle, méthyl-1-propène-1-yle, méthyl-1 propène-2-yle, méthyl-2 propène-1-yle ou méthyl-2 propène-2-yle ;

— les substituants $R_2$, identiques ou différents, représentent :

un groupement hydroxyle ;

un radical alkyle linéaire ou ramifié ayant de 1 à 4 atomes de carbone tel que ceux indiqués précédemment ;

un radical alkényle linéaire ou ramifié ayant 2 à 4 atomes de carbone tel que vinyle ou l'un de ceux indiqués précédemment ;

un radical $OR_1$, $R_1$ ayant les significations indiquées précédemment ;

un radical phényle ;

un radical cyclohexyle ;

un radical phénylalkyle dans lequel la chaîne aliphatique comporte de 1 à 3 atomes de carbone tel que benzyle, phénéthyle, phénylpropyle et phénylisopropyle ;

un radical cyclohexylalkyle dans lequel la chaîne aliphatique comporte de 1 à 3 atomes de carbone ;

un atome de chlore ou un atome de brome ;

un groupement nitro ;

un groupement aldéhyde ;

un groupement hydroxycarbonyle ;

un groupement nitrile ;

un groupement acyle de formule $—CO—R_3$ dans laquelle $R_3$ est un radical alkyle ayant de 1 à 4 atomes de carbone, un radical cyclohexyle, un radical phényle ou un enchaînement de plusieurs de ces radicaux ;

un groupement alkoxycarbonyle de formule $—COOR_4$ dans laquelle $R_4$ est un radical alkyle ayant de 1 à 4 atomes de carbone, alkényle ayant de 2 à 4 atomes de carbone, un radical cyclohexyle, phényle ou un enchaînement de plusieurs de ces radicaux ;

— n est un nombre entier de 0 à 3.

A titre d'exemples de tels éthers aryl-aliphatiques on peut citer :

— des monoéthers tels que l'anisole, l'éthoxybenzène (phénétole), le butoxybenzène, l'isobutoxybenzène, le méthoxy-1 naphtalène, l'éthoxy-2 naphtalène, le gaïacol, le méthoxy-3 phénol, le méthoxy-4 phénol, le guétol, l'éthoxy-3 phénol, l'éthoxy-4 phénol, le chloro-2 anisole, le chloro-3 anisole, le chloro-4 anisole, le bromo-2 anisole, le bromo-3 anisole, le bromo-4 anisole, le nitro-2 anisole, le nitro-3 anisole, le nitro-4 anisole, le méthyl-2 anisole, le méthyl-3 anisole, le méthyl-4 anisole, l'éthyl-2 anisole, l'éthyl-3 anisole, l'éthyl-4 anisole, l'isopropyl-2 anisole, l'isopropyl-3 anisole, l'isopropyl-4 anisole, le propyl-2 anisole, le propyl-3 anisole, le propyl-4 anisole, le (propène-1-yle)-4 anisole, l'allyl-2 anisole, l'allyl-4 anisole, le butyl-3 anisole, le butyl-4 anisole, l'isobutyl-4 anisole, le tertiobutyl-2 anisole, le tertiobutyl-3 anisole, le tertiobutyl-4 anisole, le benzyl-2 anisole, le benzyl-4 anisole, le cyclohexyl-2 anisole, le bromo-1 éthoxy-2 benzène, le bromo-1 éthoxy-3 benzène, le bromo-1 éthoxy-4 benzène, le chloro-1 éthoxy-2 benzène, le chloro-1 éthoxy-3 benzène, le chloro-1 éthoxy-4 benzène, l'éthoxy-1 nitro-2 benzène, l'éthoxy-1 nitro-3 benzène, l'éthoxy-1 nitro-4 benzène, l'éthoxy-1 éthyl-2 benzène, l'éthoxy-1 éthyl-3 benzène, l'éthoxy-1 éthyl-4 benzène, le méthoxy-2 benzaldéhyde, le méthoxy-3 benzaldéhyde, le méthoxy-4 benzaldéhyde (para-anisaldéhyde), l'éthoxy-2 benzaldéhyde, l'éthoxy-3 benzaldéhyde, l'éthoxy-4 benzaldéhyde, le dichloro-2,4 anisole, le dichloro-2,4 éthoxy-1 benzène, le méthoxy-3 pyrocatéchol, le méthoxy-2 résorcinol, le méthoxy-5 résorcinol, l'allyl-4 méthoxy-2 phénol, le diméthyl-2,3 anisole, le diméthyl-3,4 anisole, le diméthyl-2,4 anisole, le diméthyl-2,5 anisole, le dinitro-2,3 anisole, le dinitro-3,4 anisole, le dinitro-3,5 anisole, le dinitro-2,6 anisole, le dinitro-2,4 anisole, le dinitro-2,5 anisole, le dinitro-1,3 éthoxy-2 benzène, le dinitro-1,3 éthoxy-5 benzène, le dinitro-1,4 éthoxy-2 benzène, le dinitro-2,4 éthoxy-1 benzène, le chloro-3 nitro-2 anisole, le chloro-4 nitro-2 anisole, l'éthoxy-3 hydroxy-2 benzaldéhyde, l'éthoxy-3 hydroxy-4 benzaldéhyde, l'éthoxy-4 hydroxy-2 benzaldéhyde, l'éthoxy-5 hydroxy-2 benzaldéhyde, l'hydroxy-2 méthoxy-3 benzaldéhyde, l'hydroxy-2 méthoxy-4 benzaldéhyde, l'hydroxy-3 méthoxy-4 benzaldéhyde, l'hydroxy-3 méthoxy-2 benzaldéhyde, l'hydroxy-4 méthoxy-3 benzaldéhyde (vanilline), la bromo-5 vanilline, la chloro-5 vanilline, l'hydroxy-4 méthoxy-5 nitro-2 benzaldéhyde, l'hydroxy-5 méthoxy-4 nitro-2 benzaldéhyde,

— des diéthers comme le vératrole, le diméthoxy-1,3 benzène, le diméthoxy-1,4 benzène, le diéthoxy-1,2 benzène, le diéthoxy-1,3 benzène, le diéthoxy-1,4 benzène, le dipropoxy-1,2 benzène, le dipropoxy-1,3 benzène, le diméthoxy-1,2 nitro-3 benzène, le diméthoxy-1,2 nitro-4 benzène, le diméthoxy-1,3 nitro-2 benzène, le diméthoxy-1,3 nitro-5 benzène, le diméthoxy-1,4 nitro-2 benzène, le diméthoxy-2,4 nitro-1 benzène, l'allyl-1 diméthoxy-3,4 benzène, le diméthoxy-1,4 isopropyl-2 benzène, le diméthoxy-3,4 benzaldéhyde, l'éthoxy-4 méthoxy-3 benzaldéhyde, le diéthoxy-2,3 benzaldéhyde, le diéthoxy-3,4 benzaldéhyde, le diméthoxy-2,4 benzaldéhyde, le diméthoxy-1,2 dinitro-4,5 benzène, le diméthoxy-3,4 hydroxy-6 benzaldéhyde, le diméthoxy-2,6 hydroxy-4 benzaldéhyde, le diméthoxy-3,4 hydroxy-5 benzaldéhyde, le diméthoxy-3,5 hydroxy-4 benzaldéhyde (syringaldéhyde) ;

— des triéthers comme le triméthoxy-1,2,3 benzène, le triméthoxy-1,3,5 benzène, le triméthoxy-1,2,4 benzène, le triéthoxy-1,3,5 benzène, le triméthoxy-2,4,6 benzaldéhyde, le triméthoxy-3,4,5 benzaldéhyde.

Le procédé d'hydrolyse selon l'invention s'applique de manière préférée aux éthers arylaliphatiques de formule générale (I) dans laquelle :

— Ar est un cycle benzénique ;

— $R_1$ représente un radical méthyle ;

— les substituants $R_2$, identiques ou différents, représentent :

un groupement hydroxyle ;

un radical alkyle linéaire ou ramifié ayant de 1 à 4 atomes de carbone ;

un radical alkényle linéaire ou ramifié ayant de 2 à 4 atomes de carbone ;

un groupement méthoxy ;

un atome de chlore ou de brome ;

un groupement nitro ;

un groupement aldéhyde ;

— n est un nombre entier de 0 à 3.

Les composés auxquels s'applique de manière plus particulièrement intéressante le procédé selon l'invention sont : le vératrole, le gaïacol, la vanilline, l'hydroxy-3 méthoxy-4 benzaldéhyde, l'hydroxy-3 méthoxy-2 benzaldéhyde, le triméthoxy-1,2,3 benzène, le triméthoxy-1,3,5 benzène, le triméthoxy-1,2,4 benzène, le triméthoxy-2,4,6 benzaldéhyde, le triméthoxy-3,4,5 benzaldéhyde, le diméthoxy-1,3 benzène, le diméthoxy-1,4 benzène, l'anisole, le parachloroanisole, l'orthochloroanisole, le métachloroanisole, le para-anisaldéhyde, le méthoxy-2 benzaldéhyde, le méthoxy-3 benzaldéhyde, le diméthoxy-3,5 hydroxy-4 benzaldéhyde, le diméthoxy-2,3 benzaldéhyde, le diméthoxy-3,4 benzaldéhyde et le diméthoxy-3,4 hydroxy-5 benzaldéhyde.

Il est bien entendu que le procédé selon l'invention s'applique tout aussi bien aux éthers aryl-aliphatiques pris isolément qu'à des mélanges de plusieurs éthers aryl-aliphatiques.

La concentration de l'éther arylaliphatique dans le milieu réactionnel n'est pas critique et varie très largement.

On peut également opérer en masse, l'éther étant à l'état liquide et l'eau n'ayant alors que le rôle de réactif.

Si l'on exprime la concentration de l'éther par rapport au milieu réactionnel total, on opère généralement avec de 1 % à 90 % en poids d'éther. Le plus fréquemment cette concentration est comprise entre 2 % et 80 % en poids.

Lorsque l'on souhaite opérer à l'état de dissolution au moins partielle de l'éther et que celui-ci est peu soluble dans l'eau, on peut améliorer sa solubilité par l'addition d'un tiers solvant, miscible à l'eau, dans lequel l'éther est soluble.

Le procédé selon l'invention nécessite pour sa mise en œuvre un chauffage des réactifs ; la température à laquelle on opère peut varier de 150 °C à 350 °C. Elle se situe de préférence entre 220 °C et 300 °C.

La pression n'est pas un paramètre critique de la réaction. Habituellement elle est constituée par la pression autogène obtenue par chauffage, à la température désirée, du mélange réactionnel dans un appareillage adéquat fermé. Elle se situe généralement entre 10 bars et 100 bars. Mais elle peut atteindre des valeurs plus élevées car il est possible, sans sortir du cadre de l'invention, de créer dans l'appareil utilisé pour la réaction, une pression initiale à froid supérieure à la pression atmosphérique, par exemple au moyen d'un gaz inerte tel que l'azote.

L'appareillage utilisé n'est pas spécifique au procédé de l'invention. Simplement il doit présenter

certaines caractéristiques : il doit pouvoir résister aux pressions que l'on atteint lors du chauffage, il doit être étanche et évidemment ne pas être attaqué par les réactifs utilisés.

Afin d'augmenter la cinétique de la réaction d'hydrolyse et donc d'augmenter le taux de transformation pour une durée donnée de réaction, il est avantageux d'opérer l'hydrolyse en présence d'un acide carboxylique libre, qui peut être commodément l'acide correspondant au carboxylate métallique utilisé.

La quantité d'acide carboxylique libre utilisé n'est pas critique.

Lorsque l'on applique le procédé d'hydrolyse selon l'invention à un composé comportant plus d'une fonction éther, il peut être souhaitable d'hydrolyser préférentiellement une seule fonction éther.

Il est intéressant dans un tel cas d'opérer en présence de l'alcool aliphatique correspondant à la fonction éther. C'est ainsi que dans le cas des éthers méthyliques, on introduit du méthanol dans le milieu réactionnel.

La quantité d'alcool ainsi ajoutée varie largement. Plus la quantité d'alcool est importante par rapport à l'éther, plus on favorise l'hydrolyse d'une seule fonction éther par rapport à l'hydrolyse de toutes les fonctions éther du substrat.

Pratiquement le procédé selon l'invention peut être mis en œuvre de la manière suivante : on charge les différents constituants du mélange réactionnel, tels que définis précédemment, dans l'appareillage adéquat. On chauffe à la température désirée, de préférence sous agitation mais sans que celà soit véritablement indispensable, pendant une durée pouvant varier de quelques minutes à plus de 20 heures par exemple. Cependant cette durée est généralement de l'ordre de quelques heures, par exemple de 2 heures à 10 heures selon la température à laquelle on opère.

En fin de réaction, l'appareil est refroidi et la masse réactionnelle finale est traitée de manière classique, selon les réactifs utilisés ; les composés organiques autres que le sel d'acide carboxylique sont extraits par un solvant non miscible à l'eau.

Les produits obtenus sont séparés, notamment de l'éther aryl-aliphatique n'ayant pas été transformé, par des opérations courantes dans le domaine de la chimie, puis dosés si nécessaire, également par des méthodes bien connues de l'homme du métier.

Le procédé selon l'invention a de très nombreuses applications possibles, puisqu'il permet de transformer une fonction éther d'un composé aromatique en fonction phénolique.

Il présente le grand avantage de pouvoir être utilisé sur des éthers en présence d'autres composés non éthérifiés. C'est ainsi qu'on peut l'appliquer par exemple au vératrole, obtenu par méthylation du pyrocatéchol selon le procédé décrit dans la demande de brevet n° 80/18 723, et contenant du pyrocatéchol non transformé.

Il permet par exemple d'obtenir notamment du gaïacol, composé utilisé dans l'industrie pharmaceutique et servant également comme intermédiaire dans la synthèse de la vanilline, à partir du vératrole, composé ayant actuellement beaucoup moins d'applications industrielles.

Les exemples qui suivront illustrent l'invention sans en limiter la portée. Sauf mention contraire les dosages y sont effectués par chromatographie gaz-liquide.

## Exemple 1

Dans un tube de verre résistant à la pression on introduit les réactifs suivants :

| | |
|---|---|
| — acétate de sodium anhydre | 2,3 g |
| — acide acétique | 0,38 g |
| — eau distillée | 10 $cm^3$ |
| — anisole | 2,0 g |

Le tube est scellé, puis chauffé sous agitation à 250 °C et maintenu 4 heures à cette température.

Le mélange réactionnel final est incolore. Il est refroidi, puis est traité par l'éther isopropylique. L'anisole non transformé et le phénol formé sont dosés. On trouve :

— anisole non transformé 1,871 g soit un taux de transformation (T.T.) de 6,5 %.
— phénol formé 0,113 g soit un rendement par rapport à l'anisole transformé (R.T.) de 100 %.

## Exemple 2

On utilise le même mode opératoire que dans l'exemple 1. On introduit les réactifs suivants :

| | |
|---|---|
| — acétate de sodium anhydre | 2,3 g |
| — acide acétique | 0,38 g |
| — eau distillée | 8 $cm^3$ |
| — méthanol | 2 $cm^3$ |
| — anisole | 2,0 g |

Le mélange réactionnel final est incolore. Il est traité et dosé comme dans l'exemple 1.

| | |
|---|---|
| — T.T. de l'anisole : | 6,6 % |
| — R.T. en phénol : | 100 % |

Exemple 3

On utilise le même mode opératoire que dans l'exemple 1. On introduit les réactifs suivants :

| | |
|---|---|
| — acétate de sodium anhydre | 4,6 g |
| — acide acétique | 0,38 g |
| — eau distillée | 10 $cm^3$ |
| — anisole | 2,0 g |

Le mélange réactionnel final est incolore. Il est traité et dosé comme dans l'exemple 1.

| | |
|---|---|
| — T.T. de l'anisole : | 6,6 % |
| — R.T. en phénol : | 100 % |

Exemple 4

On utilise le même mode opératoire que dans l'exemple 1. On introduit les réactifs suivants :

| | |
|---|---|
| — acétate de sodium anhydre | 2,3 g |
| — acide acétique | 0,76 g |
| — eau distillée | 10 $cm^3$ |
| — anisole | 2,0 g |

Le mélange réactionnel final est incolore. Il est traité et dosé comme dans l'exemple 1.

| | |
|---|---|
| — T.T. de l'anisole : | 8,1 % |
| — R.T. en phénol : | 100 % |

Exemple 5

On utilise le même mode opératoire que dans l'exemple 1. On introduit les réactifs suivants :

| | |
|---|---|
| — acétate de sodium anhydre | 2,3 g |
| — eau distillée | 10 $cm^3$ |
| — anisole | 2,0 g |

Le mélange réactionnel final est incolore. Il est traité et dosé comme dans l'exemple 1.

| | |
|---|---|
| — T.T. de l'anisole : | 6,3 % |
| — R.T. en phénol : | 100 % |

Exemple 6

On utilise le même mode opératoire que dans l'exemple 1. On introduit les réactifs suivants :

| | |
|---|---|
| — acétate de sodium anhydre | 2,3 g |
| — acide acétique | 0,38 g |
| — eau distillée | 10 $cm^3$ |
| — parachloroanisole | 2,0 g |

Le mélange réactionnel final est incolore. Il est traité et dosé comme dans l'exemple 1.

| | |
|---|---|
| — T.T. du parachloroanisole : | 12,8 % |
| — R.T. en parachlorophénol : | 85 % |

Exemple 7

On utilise le même mode opératoire que dans l'exemple 1. La durée de chauffage à 250° est 2 heures au lieu de 4 heures. On introduit les réactifs suivants :

| | |
|---|---|
| — acétate de sodium anhydre | 2,3 g |
| — acide acétique | 0,38 g |

| | |
|---|---|
| — eau distillée | 5 $cm^3$ |
| — méthanol | 5 $cm^3$ |
| — vératrole | 2,0 g |

Le mélange réactionnel final est incolore. Il est traité et dosé comme dans l'exemple 1.

| | |
|---|---|
| — T.T. du vératrole : | 22,9 % |
| — R.T. en gaïacol : | 90,0 % |
| — R.T. en pyrocatéchol : | 10,0 % |

Exemple 8

On utilise le même mode opératoire que dans l'exemple 1. La durée de chauffage à 250° est 5 heures au lieu de 4 heures. On introduit les réactifs suivants :

| | |
|---|---|
| — acétate de sodium anhydre | 2,3 g |
| — acide acétique | 0,38 g |
| — eau distillée | 5 $cm^3$ |
| — méthanol | 5 $cm^3$ |
| — vératrole | 2,0 g |

Le mélange réactionnel final est incolore. Il est traité et dosé comme dans l'exemple 1.

| | |
|---|---|
| — T.T. du vératrole : | 48,0 % |
| — R.T. en gaïacol : | 75,0 % |
| — R.T. en pyrocatéchol : | 25 % |

Exemple 9

On utilise le même mode opératoire que dans l'exemple 1. La durée de chauffage à 250° est 2 heures au lieu de 4 heures. On introduit les réactifs suivants :

| | |
|---|---|
| — acétate de sodium anhydre | 2,3 g |
| — acide acétique | 0,38 g |
| — eau distillée | 10 $cm^3$ |
| — vératrole | 2,0 g |

Le mélange réactionnel final est incolore. Il est traité et dosé comme dans l'exemple 1.

| | |
|---|---|
| — T.T. du vératrole : | 22,6 % |
| — R.T. en gaïacol : | 90,0 % |
| — R.T. en pyrocatéchol : | 10,0 % |

Exemple 10

On utilise le même mode opératoire que dans l'exemple 1. La durée de chauffage à 250° est 5 heures au lieu de 4 heures. On introduit les réactifs suivants :

| | |
|---|---|
| — acétate de sodium anhydre | 2,3 g |
| — acide acétique | 0,38 g |
| — eau distillée | 10 $cm^3$ |
| — vératrole | 2,0 g |

Le mélange réactionnel final est incolore. Il est traité et dosé comme dans l'exemple 1.

| | |
|---|---|
| — T.T. du vératrole : | 55,5 % |
| — R.T. en gaïacol : | 63,0 % |
| — R.T. en pyrocatéchol : | 37,0 % |

**Revendications**

1. Procédé l'hydrolyse d'au moins une fonction éther d'un composé de formule générale (I) :

$$R_1O—Ar—(R_2)_n \qquad (I)$$

dans laquelle :

— Ar représente un cycle benzénique ou un cycle naphtalénique,

— $R_1$ représente :

un radical alkyle linéaire ou ramifié ayant de 1 à 4 atomes de carbone,

un radical alkényle linéaire ou ramifié ayant 3 ou 4 atomes de carbone,

— Les substituants $R_2$ identiques ou différents, représentent :

un groupement hydroxyle ;

un radical $OR_1$, $R_1$ ayant les significations indiquées précédemment ;

un radical alkyle linéaire ou ramifié ayant de 1 à 6 atomes de carbone ;

un radical alkényle linéaire ou ramifié ayant de 2 à 6 atomes de carbone ;

un radical phényle ;

un radical cycloalkyle ;

un radical phénylalkyle dans lequel la chaîne aliphatique comporte de 1 à 4 atomes de carbone ;

un radical cycloalkylalkyle dans lequel la chaîne aliphatique comporte de 1 à 4 atomes de carbone ;

un atome d'halogène ;

un groupement nitro ;

un groupement amine ;

un groupement aldéhyde —CHO ;

un groupement nitrile ;

un groupement hydroxycarbonyle ;

un groupement acyle de formule —CO—$R_3$ dans laquelle $R_3$ est un radical alkyle ayant de 1 à 6 atomes de carbone, cycloalkyle ayant 5 ou 6 atomes de carbone, phényle, ou un enchaînement de plusieurs de ces radicaux ;

un groupement alkoxycarbonyle de formule —$COOR_4$ dans laquelle $R_4$ est un radical alkyle ayant de 1 à 6 atomes de carbone, alkényle ayant de 2 à 6 atomes de carbone, cycloalkyle ayant 5 ou 6 atomes de carbone, phényle ou un enchaînement de plusieurs de ces radicaux ;

— n est un nombre entier de 0 à 5,

ledit procédé étant caractérisé en ce que l'on opère en présence d'eau et en présence d'une quantité efficace d'un sel d'acide carboxylique choisi parmi les carboxylates d'un métal alcalin, d'ammonium ou d'un métal alcalino-terreux.

2. Procédé selon la revendication 1, caractérisé en ce que le sel d'acide carboxylique est un carboxylate de métal alcalin.

3. Procédé selon l'une quelconque des revendications 1 à 2, caractérisé en ce que le sel d'acide carboxylique utilisé est un sel d'un acide aliphatique saturé monofonctionnel ayant de 2 à 6 atomes de carbone ou difonctionnel ayant de 3 à 6 atomes de carbone, de l'acide benzoïque ou de l'un des acides ortho-, méta- ou téréphtaliques.

4. Procédé selon l'une quelconque des revendications 1 à 3, caractérisé en ce qu'il est appliqué à un composé de formule générale (I) :

$$R_1O—Ar—(R_2)_n \qquad (I)$$

dans laquelle :

— Ar représente un cycle benzénique ou un cycle naphtalénique,

— $R_1$ représente :

un radical alkyle linéaire ou ramifié ayant de 1 à 4 atomes de carbone,

un radical alkényle linéaire ou ramifié ayant 3 ou 4 atomes de carbone,

— Les substituants $R_2$ identiques ou différents, représentent :

un groupement hydroxyle ;

un radical alkyle linéaire ou ramifié ayant de 1 à 4 atomes de carbone ;

un radical alkényle linéaire ou ramifié ayant 2 à 4 atomes de carbone ;

un radical $OR_1$, $R_1$ ayant les significations indiquées précédemment ;

un radical phényle ;

un radical cyclohexyle ;

un radical phénylalkyle dans lequel la chaîne aliphatique comporte de 1 à 3 atomes de carbone ;

un radical cyclohexylalkyle dans lequel la chaîne aliphatique comporte de 1 à 3 atomes de carbone ;

un atome de chlore ou un atome de brome ;

un groupement nitro ;

un groupement aldéhyde ;

un groupement hydroxycarbonyle ;

un groupement nitrile ;

un groupement acyle de formule —CO—$R_3$ dans laquelle $R_3$ est un radical alkyle ayant de 1 à 4 atomes de carbone, un radical cyclohexyle, un radical phényle ou un entraînement de plusieurs de ces radicaux ;

un groupement alkoxycarbonyle de formule —$COOR_4$ dans laquelle $R_4$ est un radical alkyle ayant

de 1 à 4 atomes de carbone, alkényle ayant de 2 à 4 atomes de carbone, un radical cyclohexyle, phényle ou un enchaînement de plusieurs de ces radicaux ;

— n est un nombre entier de 0 à 3.

5. Procédé selon l'une quelconque des revendications 1 à 4, caractérisé en ce qu'il est appliqué à un composé de formule générale (I) :

$$R_1O—Ar—(R_2)_n \quad (I)$$

dans laquelle :

— Ar représente un cycle benzénique ;

— $R_1$ représente un radical méthyle ;

— les substituants $R_2$, identiques ou différents, représentent :

un groupement hydroxyle ;

un radical alkyle linéaire ou ramifié ayant de 1 à 4 atomes de carbone ;

un radical alkényle linéaire ou ramifié ayant de 2 à 4 atomes de carbone ;

un groupement méthoxy ;

un atome de chlore ou de brome ;

un groupement nitro ;

un groupement aldéhyde ;

— n est un nombre entier de 0 à 3.

6. Procédé selon l'une quelconque des revendications 1 à 5, caractérisé en ce que l'on opère à une température située entre 150 °C et 350 °C, et de préférence entre 220 °C et 300 °C.

7. Procédé selon l'une quelconque des revendications 1 à 6, caractérisé en ce que l'on utilise un rapport pondéral sel d'acide carboxylique/éther aryl-aliphatique de 0,01 à 50 et de préférence de 0,05 à 20.

8. Procédé selon l'une quelconque des revendications 1 à 7, caractérisé en ce que l'on opère en présence d'acide carboxylique libre.

9. Procédé selon l'une quelconque des revendications 1 à 8 appliqué à un éther aryl-aliphatique ayant plus d'une fonction éther caractérisée en ce que l'on opère en présence de l'alcool correspondant aux fonctions éther afin d'hydrolyser préférentiellement une desdites fonctions éther.

## Claims

1. A process for the hydrolysis of at least one ether group of a compound having the general formula (I) :

$$R_1O—Ar—(R_2)_n \quad (I)$$

in which :

— Ar represents a benzene ring or a naphthalene ring,

— $R_1$ represents :

a linear or branched alkyl radical having 1 to 4 carbon atoms, or

a linear or branched alkenyl radical having 3 or 4 carbon atoms,

— the substituents $R_2$, which may be identical or different, represent :

a hydroxyl group,

a $OR_1$ radical, $R_1$ having the meanings previously indicated,

a linear or branched alkyl radical having 1 to 6 carbon atoms,

a linear or branched alkenyl radical having 2 to 6 carbon atoms,

a phenyl radical,

a cycloalkyl radical,

a phenylalkyl radical, in which the aliphatic chain contains 1 to 4 carbon atoms,

a cycloalkylalkyl radical, in which the aliphatic chain contains 1 to 4 carbon atoms,

a halogen atom,

a nitro group,

a amino group,

an aldehyde group —CHO,

a nitrile group,

a hydroxycarbonyl group,

an acyl group having the formula $—CO—R_3$, in which $R_3$ is an alkyl radical having 1 to 6 carbon atoms, a cycloalkyl radical radical having 5 or 6 carbon atoms, a phenyl radical or a chain of several of these radicals, or

an alkoxycarbonyl group having the formula $—COOR_4$, in which $R_4$ is an alkyl radical having 1 to 6 carbon atoms, an alkenyl radical having 2 to 6 carbon atoms, a cycloalkyl radical having 5 or 6 carbon atoms, a phenyl radical or a chain of several of these radicals, an n is and integer from 0 to 5, the process being characterised in that it is carried out in the presence of water and in the presence of an

effective amount of a carboxylic acid salt chosen among the carboxylates of an alkali metal, of ammonium or of an alkaline earth metal.

2. A process according to claim 1, characterised in that the carboxylic acid salt is an alkali metal carboxylate.

3. A process according to any one of claims 1 and 2, characterised in that the carboxylic acid salt used is a salt of a monofunctional satured aliphatic acid having 2 to 6 carbon or difunctional saturated aliphatic acid having 3 to 6 carbon atoms, of benzoic acid or of ortho-, meta- or terephthalic acid.

4. A process according to any one of claims 1 to 3, characterised in that it is applied to a compound having the general formula (I) :

$$R_1O—Ar—(R_2)_n \quad (I)$$

in which :

— Ar represents a benzene ring or a naphthalene ring,

— $R_1$ represents :

a linear or branched alkyl radical having 1 to 4 carbon atoms, or

a linear or branched alkenyl radical having 3 or 4 carbon atoms,

— the substituents $R_2$, which may be identical or different, represent :

a hydroxyl group,

a linear or branched alkyl radical having 1 to 4 carbon atoms,

a linear or branched alkenyl radical having 2 to 4 carbon atoms,

an $OR_1$ radical, $R_1$ having the meanings previously indicated,

a phenyl radical,

a cyclohexyl radical,

a phenylalkyl radical, in which the aliphatic chain contains 1 to 3 carbon atoms,

a cyclohexylalkylradical, in which the aliphatic chain contains 1 to 3 carbon atoms,

a chlorine atom or a bromine atom,

a nitro group,

an aldehyde group,

a hydroxycarbonyl group,

a nitrile group,

an acyl group having the formula $—CO—R_3$, in which $R_3$ is an alkyl radical having 1 to 4 carbon atoms, a cyclohexyl radical, a phenyl radical or a chain of several of these radicals, or an alkoxycarbonyl group having the formula $—COOR_4$, in which $R_4$ is an alkyl radical having 1 to 4 carbon atoms, an alkenyl radical having 2 to 4 carbon atoms, a cyclohexyl radical, a phenyl radical or a chain of several of the radicals, and

— n is an integer from 0 to 3.

5. A process according to any one of claims 1 and 4 characterised in that it is applied to a compound having the general formula (I) :

$$R_1O—Ar(R_2)_n \quad (I)$$

in which :

— Ar represents a benzene ring,

— $R_1$ represents a methyl radical,

— the substituents $R_2$, which may be identical or different, represent :

a hydroxyl group,

a linear or branched alkyl radical having 1 to 4 carbon atoms,

a linear or branched alkenyl radical having 2 to 4 carbone atoms,

a methoxy group,

a chlorine atom or bromine atom,

a nitro group, or

an aldehyde group, and

— n is an, integer from 0 to 3.

6. A process according to any one of claims 1 to 5, characterised in that it is carried out at a temperature of between 150 °C and 350 °C and preferably between 220 °C and 300 °C.

7. A process according to any one of claims 1 to 6, characterised in that a weight ratio of carboxylic acid salt to aryl-aliphatic ether of from 0.01 to 50 and preferably from 0.05 to 20 is used.

8. A process according to any one of claims 1 to 7, characterised in that it is carried out in the presence of free carboxylic acid.

9. A process according to any one of claims 1 to 8, applied to an aryl-aliphatic ether having more than one ether group, characterised in that it is carried out in the presence of the alcohol corresponding to the ether groups, so as to hydrolyse preferentially one of the said ether groups.

**Patentansprüche**

1. Verfahren zur Hydrolyse zumindest einer Ätherfunktion einer Verbindung der allgemeinen Formel (I):

$$R_1O—Ar—(R_2)_n \quad (I)$$

in welcher:

— Ar einen Benzolring oder einen Naphthalinring darstellt,

— $R_1$ darstellt:

einen geradkettigen oder verzweigten Alkylrest mit 1 bis 4 Kohlenstoffatomen,

einen geradkettigen oder verzweigten Alkenylrest mit 3 oder 4 Kohlenstoffatomen,

— die Substituenten $R_2$, die gleich oder voneinander verschieden sind, darstellen:

eine Hydroxylgruppe,

eine Rest $OR_1$, wobei $R_1$ die zuvor angegebenen Bedeutungen hat;

einen geradkettigen oder verzweigten Alkylrest mit 1 bis 6 Kohlenstoffatomen;

einen geradkettigen oder verzweigten Alkenylrest mit 2 bis 6 Kohlenstoffatomen;

eine Phenylrest;

einen Cycloalkylrest;

eine Phenylalkylrest, worin die aliphatische Kette 1 bis 4 Kohlenstoffatome enthält;

einen Cycloalkylalkylrest, worin die aliphatische Kette 1 bis 4 Kohlenstoffatome enthält;

eine Halogenatom;

eine Nitrogruppe;

eine Amingruppe;

eine Aldehydgruppe —CHO;

eine Nitrilgruppe;

eine Hydroxycarbonylgruppe;

eine Acylgruppe der Formel $—CO—R_3$, in welcher $R_3$ ein Alkylrest mit 1 bis 6 Kohlenstoffatomen, ein Cycloalkylrest mit 5 oder 6 Kohlenstoffatomen ein Phenylrest oder eine Gruppierung von mehreren dieser Reste ist;

eine Alkoxycarbonylgruppe der Formel $—COOR_4$, in welcher $R_4$ ein Alkylrest mit 1 bis 6 Kohlenstoffatomen, ein Alkenylrest mit 2 bis 6 Kohlenstoffatomen, ein Cycloalkylrest mit 5 oder 6 Kohlenstoffatomen, ein Phenylrest oder eine Gruppierung mehrerer dieser Reste, ist;

— n eine ganze Zahl von 0 bis 5 ist,

welches Verfahren dadurch gekennzeichnet ist, daß man in Gegenwart von Wasser und in Gegenwart einer wirksamen Menge eines Carbonsäuresalzes, ausgewählt aus den Carboxylaten eines Alkalimetalls, von Ammonium oder eines Erdalkalimetalls, arbeitet.

2. Verfahren nach Anspruch 1, dadurch gekennzeichnet, daß das Carbonsäuresalz ein Alkalimetallcarboxylat ist.

3. Verfahren nach einem der Ansprüche 1 bis 2, dadurch gekennzeichnet, daß das verwendete Carbonsäuresalz ein Salz einer gesättigten aliphatischen Säure, die einwertig ist und 2 bis 6 Kohlenstoffatome enthält oder zweiwertig ist und 3 bis 6 Kohlenstoffatome enthält, von Benzoesäure oder einer der Ortho-, Meta- oder Terephthalsäuren ist.

4. Verfahren nach einem der Ansprüche 1 bis 3, dadurch gekennzeichnet, daß es auf eine Verbindung der allgemeinen Formel (I):

$$R_1O—Ar—(R_2)_n \quad (I)$$

angewendet wird, in welcher:

— Ar einen Benzolring oder einen Naphthalinring darstellt,

— $R_1$ darstellt:

einen geradkettigen oder verzweigten Alkylrest mit 1 bis 4 Kohlenstoffatomen,

einen geradkettigen oder verzweigten Alkenylrest mit 3 oder 4 Kohlenstoffatomen,

— die Substituenten $R_2$, unabhängig voneinander darstellen:

eine Hydroxylgruppe;

eine geradkettigen oder verzweigten Alkylrest mit 1 bis 4 Kohlenstoffatomen;

einen geradkettigen oder verzweigten Alkenylrest mit 2 bis 4 Kohlenstoffatomen;

einen Rest $OR_1$, wobei $R_1$ die oben angegebenen Bedeutungen hat;

einen Phenylrest;

einen Cyclohexylrest;

einen Phenylalkylrest, worin die aliphatische Kette 1 bis 3 Kohlenstoffatome enthält;

einen Cyclohexylalkylrest, worin die aliphatische Kette 1 bis 3 Kohlenstoffatome enthält;

ein Chloratom oder ein Bromatom;

eine Nitrogruppe;

eine Aldehydgruppe;

eine Hydroxycarbonylgruppe ;
eine Nitrilgruppe ;
eine Acylgruppe der Formel —CO—$R_3$, in welcher $R_3$ ein Alkylrest mit 1 bis 4 Kohlenstoffatomen, ein Cyclohexylrest, ein Phenylrest oder eine Gruppierung von mehreren dieser Reste ist ;
eine Alkoxycarbonylgruppe der Formel —COO$R_4$, worin $R_4$ ein Alkylrest mit 1 bis 4 Kohlenstoffatomen, ein Alkenylrest mit 2 bis 4 Kohlenstoffatomen, ein Cyclohexylrest, Phenylrest oder eine Gruppierung von mehreren dieser Reste ist ;
— n eine ganze Zahl von 0 bis 3 ist.

5. Verfahren nach einem der Ansprüche 1 bis 4, dadurch gekennzeichnet, daß es auf eine Verbindung der allgemeinen Formel (I) :

$$R_1C—Ar—(R_2)_n \qquad (I)$$

angewendet wird, in welcher :
— Ar einen Benzolring darstellt ;
— $R_1$ einen Methylrest darstellt ;
— die Substituenten $R_2$ unabhängig voneinander darstellen :
eine Hydroxylgruppe ;
einen geradkettigen oder verzweigten Alkylrest mit 1 bis 4 Kohlenstoffatomen ;
einen geradkettigen oder verzweigten Alkenylrest mit 2 bis 4 Kohlenstoffatomen ;
eine Methoxygruppe ;
ein Chlor- oder Bromatom ;
eine Nitrogruppe ;
eine Aldehydgruppe ;
— n eine ganze Zahl von 0 bis 3 ist.

6. Verfahren nach einem der Ansprüche 1 bis 5, dadurch gekennzeichnet, daß man bei einer Temperatur zwischen 150 und 350 °C, vorzugsweise zwischen 220 °C und 300 °C, arbeitet.

7. Verfahren nach einem der Ansprüche 1 bis 6, dadurch gekennzeichnet, daß man ein Gewichtsverhältnis von Carbonsäuresalzarylaliphatischem Äther von 0,01 bis 50, vorzugsweise von 0,05 bis 20, verwendet.

8. Verfahren nach einem der Ansprüche 1 bis 7, dadurch gekennzeichnet, daß man in Gegenwart von freier Carbonsäure arbeitet.

9. Verfahren nach einem der Ansprüche 1 bis 8, angewendet auf einen aryl-aliphatischen Äther, der mehr als eine Ätherfunktion aufweist, dadurch gekennzeichnet, daß man in Gegenwart des den Ätherfunktionen entsprechenden Alkohols arbeitet, um vorzugsweise eine der erwähnten Ätherfunktionen zu hydrolysieren.